(19) Europäisches Patentamt
European Patent Office
Office européen des brevets

(11) EP 4 151 304 A1

(12) EUROPEAN PATENT APPLICATION
published in accordance with Art. 153(4) EPC

(43) Date of publication:
22.03.2023 Bulletin 2023/12

(21) Application number: 21803547.5

(22) Date of filing: 28.04.2021

(51) International Patent Classification (IPC):
*B01D 61/14 (2006.01)* *B01D 63/02 (2006.01)*
*B01D 71/48 (2006.01)* *B01D 71/68 (2006.01)*
*B01J 20/26 (2006.01)* *A61M 1/18 (2006.01)*

(52) Cooperative Patent Classification (CPC):
**B01D 61/14; B01D 63/02; B01D 71/48; B01D 71/68; B01J 20/26**

(86) International application number:
PCT/JP2021/016934

(87) International publication number:
WO 2021/230082 (18.11.2021 Gazette 2021/46)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 11.05.2020 JP 2020083505

(71) Applicant: **Nikkiso Co., Ltd.**
**Tokyo 150-6022 (JP)**

(72) Inventor: **MAEDA Hayata**
**Kanazawa-shi Ishikawa 920-0177 (JP)**

(74) Representative: **Algemeen Octrooi- en Merkenbureau B.V.**
**P.O. Box 645**
**5600 AP Eindhoven (NL)**

# (54) HOLLOW FIBER MEMBRANE MODULE AND METHOD FOR REMOVING ENDOTOXINS

(57) A hollow fiber membrane module (1) includes: a first hollow fiber membrane (16a) whose sieving coefficient for dextran having a molecular weight of not less than 300 kDa nor more than 1000 kDa is not less than 0.12 nor more than 0.28, wherein the first hollow fiber membrane (16a) adsorbs endotoxins.

FIG. 1

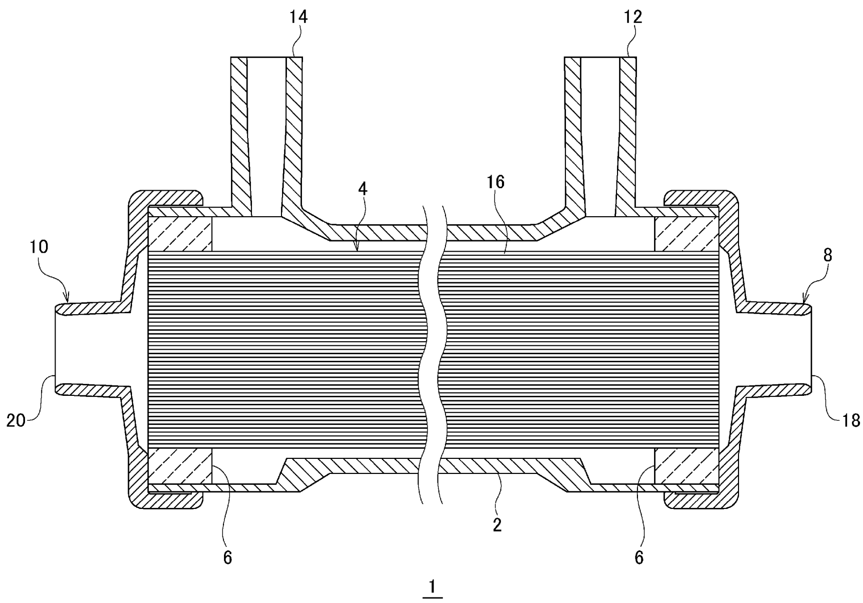




Processed by Luminess, 75001 PARIS (FR)

## Description

[TECHNICAL FIELD]

The present invention relates to hollow fiber membrane modules and a method for removing endotoxins.

[BACKGROUND ART]

In medical fields such as emergency intensive care, treatment for removing endotoxins from the body of septic patients and other patients through blood purification therapy is performed. Endotoxins are one of inflammation-inducing substances represented by pathogen-associated molecular patterns (PAMPs). As a conventional endotoxin removal technique, a method is known that adsorbs and removes endotoxins by bringing blood to come into contact directly with an immobilized polymyxin molded article in which an endotoxin adsorbent such as polymyxin B is immobilized on a carrier (see, for example, Patent Literature 1).

[Patent Literature 1] Japanese Unexamined Patent Application Publication NO. S61-135674

[SUMMARY OF INVENTION]

[TECHNICAL PROBLEM]

Patients with severe sepsis may also suffer from acute kidney injury (AKI), etc., and renal replacement therapy may be performed in another blood purifier in parallel with endotoxin removal. In this case, two modules, an endotoxin removal module and a renal replacement therapy module, need to be connected in series or parallel in the blood circuit. However, when conventional immobilized polymyxin molded articles are used as endotoxin removal modules, there is room for improvement in terms of practicality due to complication of the structure of the blood circuit, an increase in the cost of treatment, etc.

In this background, a purpose of the present invention is to provide a more practical endotoxin removal technology.

[SOLUTION TO PROBLEM]

One embodiment of the present invention relates to a hollow fiber membrane module. This hollow fiber membrane module includes: a first hollow fiber membrane whose sieving coefficient for dextran having a molecular weight of not less than 300 kDa nor more than 1000 kDa is not less than 0.12 nor more than 0.28, wherein the first hollow fiber membrane adsorbs endotoxins.

Another embodiment of the present invention is a method for removing endotoxins. This method for removing endotoxins includes bringing the hollow fiber membrane module according to any one of the embodiments to come into contact with the endotoxins.

Optional combinations of the aforementioned constituting elements, and implementations of the invention in the form of methods, devices, systems, etc., may also be practiced as additional modes of the present invention.

[ADVANTAGEOUS EFFECTS OF INVENTION]

The present invention can provide a more practical endotoxin removal technology.

[BRIEF DESCRIPTION OF DRAWINGS]

FIG. 1 is a cross-sectional view of a hollow fiber membrane module according to an embodiment;
FIG. 2 is a schematic diagram for explaining the principle of removing target substances by a hollow fiber membrane;
FIG. 3A is a scanning electron micrograph of a first hollow fiber membrane;
FIG. 3B is a scanning electron micrograph of a second hollow fiber membrane;
FIG. 4 is a schematic diagram of a circulator used in a test;
FIG. 5A shows molecular weight cut off curves of respective hollow fiber membranes;
FIG. 5B shows the sieving coefficients of the respective hollow fiber membranes in the molecular weight range of endotoxins and the molecular weight range of interleukins 6;
FIGS. 6A to 6D are diagrams showing the results of endotoxin adsorption tests;

FIG. 7 is a diagram showing the calculation results of endotoxin adsorption amounts;
FIG. 8A to FIG. 8D are diagrams showing the results of interleukin 6 adsorption tests; and
FIG. 9 is a diagram showing the calculation results of interleukin 6 adsorption amounts.

[DESCRIPTION OF EMBODIMENTS]

One embodiment of the present invention that addresses the issue is a hollow fiber membrane module. This hollow fiber membrane module includes: a first hollow fiber membrane whose sieving coefficient for dextran having a molecular weight of not less than 300 kDa nor more than 1000 kDa is not less than 0.12 nor more than 0.28, wherein the first hollow fiber membrane adsorbs endotoxins.
In the above embodiment, the first hollow fiber membrane may adsorb the endotoxins in the blood. Further, the first hollow fiber membrane may be composed of polyester polymer alloy membrane containing a polyarylate resin having repeating units represented by the following chemical formula (1) and a polyethersulfone resin having repeating units represented by the following chemical formula (2) or (3):

[Chemical 1]

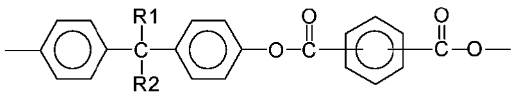

(1)

where, in the chemical formula (1), R1 and R2 are lower alkyl groups having 1 to 5 carbons, and R1 and R2 may be identical or different from each other;

[Chemical 2]

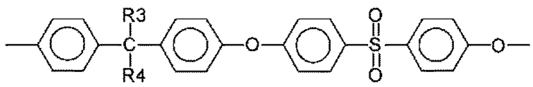

(2)

where, in the chemical formula (2), R3 and R4 are lower alkyl groups having 1 to 5 carbons, and R3 and R4 may be identical or different from each other; and

[Chemical 3]

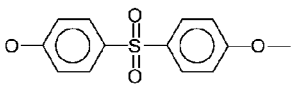

(3)

Further, the hollow fiber membrane module may include: a second hollow fiber membrane whose sieving coefficient for dextran having a molecular weight of not less than 20 kDa nor more than 30 kDa is not less than 0.64 nor more than 0.80, wherein the second hollow fiber membrane may adsorb interleukins 6.
Another embodiment of the present invention is a method for removing endotoxins. This method for removing endotoxins includes bringing the hollow fiber membrane module according to any one of the embodiments to come into contact with the endotoxins.
Hereinafter, the present invention will be described based on preferred embodiments with reference to the drawings. The embodiments do not limit the invention and are shown for illustrative purposes, and not all the features described in the embodiments and combinations thereof are necessarily essential to the invention. The same or equivalent constituting elements, members, and processes illustrated in each drawing shall be denoted by the same reference numerals, and duplicative explanations will be omitted appropriately. The scales and shapes shown in the figures are defined for convenience's sake to make the explanation easy and shall not be interpreted limitatively unless otherwise specified. Terms like "first", "second", etc., used in the specification and claims do not indicate an order or importance by any means and are used to distinguish a certain feature from the others. Some of the components in each figure may be omitted if they are not important for explanation.
FIG. 1 is a cross-sectional view of a hollow fiber membrane module according to an embodiment. A hollow

fiber membrane module 1 of the present embodiment takes, for example, a form similar to that of a dialyzer for hemodialysis (HD); however, the form is not limited to this form. The hollow fiber membrane module 1 includes a column 2, a hollow fiber membrane bundle 4, a sealing member 6, a first header 8, and a second header 10.

The column 2 is a cylindrical container that houses the hollow fiber membrane bundle 4. The column 2 is made of resin such as polycarbonate, for example. The column 2 has openings at the respective ends in the longitudinal direction. Also, the column 2 has a dialysate discharge port 12 and a dialysate introduction port 14 protruding radially from the cylinder near both ends in the longitudinal direction. A dialysate is supplied into the column 2 from the outside via the dialysate introduction port 14 and is then discharged to the outside from the inside of the column 2 via the dialysate discharge port 12. When the hollow fiber membrane module 1 is used for hemofiltration (HF), only the dialysate discharge port 12 or the dialysate introduction port 14 is provided as a filtrate discharge port.

The hollow fiber membrane bundle 4 has a structure consisting of a plurality of hollow fiber membranes 16 bundled together. Each hollow fiber membrane 16 extends approximately parallel to the longitudinal direction of the column 2. The openings at the respective ends of each hollow fiber membrane 16 are open, and blood flows from one opening to the other. The configuration of the hollow fiber membranes 16 will be explained in detail later.

The sealing member 6, also called potting material, is composed of a thermosetting resin such as polyurethane, phenolic resin, epoxy resin, etc. The sealing member 6 fills gaps between the plurality of hollow fiber membranes 16 at each end of the hollow fiber membrane bundle 4. The sealing member 6 is also fixed to the inner surface of the column 2 at each end. This causes the hollow fiber membrane bundle 4 to be fixed to the column 2 and the respective openings at both ends of the column 2 to be sealed.

The first header 8 is attached to one end of the column 2 and seals the opening at one end. The second header 10 is attached to the other end of the column 2 and seals the opening at the other end. The first header 8 has a blood introduction port 18 that connects the inside and outside of the column 2. The second header 10 has a blood discharge port 20 that connects the inside and outside of the column 2. A tube for removing blood from a patient is connected to the blood introduction port 18, and a tube for returning blood to the patient is connected to the blood discharge port 20.

Blood removed from the patient is supplied into the column 2 via the blood introduction port 18. The blood supplied into the column 2 flows through each of the hollow fiber membranes 16 and moves toward the blood discharge port 20. At this time, material exchange occurs through the hollow fiber membranes 16 between the blood flowing inside the hollow fiber membranes 16 and the dialysate flowing outside the hollow fiber membranes 16. The blood that has passed through the hollow fiber membranes 16 is discharged to the outside of the column 2 through the blood discharge port 20 and returned to the patient.

The configuration of the hollow fiber membranes 16 will be explained in detail next. Each hollow fiber membranes 16 is composed of a hydrophobic polymer porous semipermeable membrane that is mainly composed of polysulfone resin and polyester resin. Each hollow fiber membrane 16 is a thin tube having a thickness of 5 to 150 um and a circular cross section whose inner diameter of about 100 to 500 $\mu$m.

The hollow fiber membranes 16 according to the present embodiment are composed of polyester polymer alloy (PEPA) membranes containing a polyarylate resin having repeating units represented by the following chemical formula (1) and a polyethersulfone resin having repeating units represented by the following chemical formula (2) or (3). The indication of the phthalic acid moiety in chemical formula (1) means that the arrangement of two carboxyl groups relative to a benzene ring does not matter. For example, a structure in which two carboxyl groups are bonded to a benzene ring in a meta position with respect to each other (i.e., isophthalic acid) or in a para-position with respect to each other (i.e., terephthalic acid) is included.

[Chemical Formula 4]

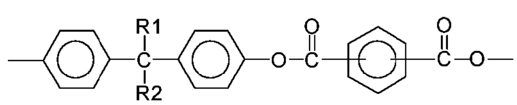

(1)

In the chemical formula (1), R1 and R2 are lower alkyl groups having 1 to 5 carbons, and R1 and R2 may be identical or different from each other.

[Chemical Formula 5]

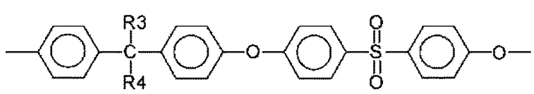

(2)

In the chemical formula (2), R3 and R4 are lower alkyl groups having 1 to 5 carbons, and R3 and R4 may be identical or different from each other.

[Chemical Formula 6]

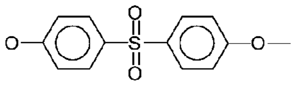

(3)

FIG. 2 is a schematic diagram for explaining the principle of removing target substances T by a hollow fiber membrane 16. There are three main principles of removing target substances T by a hollow fiber membranes 16: dialysis; filtration; and adsorption. The differences in the principles of removing target substances have a significant impact on the efficiency of removing substances with large molecular weight, such as inflammation-inducing substances. As shown in FIG. 2, the hollow fiber membrane 16 according to the present embodiment has pores 22 with diameters corresponding to the size of the target substances T and removes the target substances T by adsorption. The target substances T contained in blood flowing inside the hollow fiber membrane 16 flow toward the outside of the hollow fiber membrane 16 and enter the pores 22. Due to van der Waals forces and hydrophobic interactions that occur between the target substances T and the inner surfaces of the pores 22, the target substances T that have entered the pores 22 adhere to the inner wall surfaces. Thereby, the target substances T are efficiently collected.

The hollow fiber membrane module 1 according to the present embodiment includes a first hollow fiber membrane 16a and a second hollow fiber membrane 16b. Both the first hollow fiber membrane 16a and the second hollow fiber membrane 16b are composed of the above-mentioned PEPA membranes. FIG. 3A is a scanning electron micrograph of the first hollow fiber membrane 16a. FIG. 3B is a scanning electron micrograph of the second hollow fiber membrane 16b. As shown in FIG. 3A, the first hollow fiber membrane 16a has a plurality of first pores 22a. As shown in FIG. 3B, the second hollow fiber membrane 16b has a plurality of second pores 22b.

The target substances T in the hollow fiber membrane module 1 according to the present embodiment are endotoxins and interleukins 6. The interleukins 6 are hereafter denoted as IL-6, as appropriate. The first hollow fiber membrane 16a adsorbs endotoxins by means of the first pores 22a. Endotoxins have hydrophilic and hydrophobic segments and have a micellar structure in aqueous solution. The apparent molecular weight of the endotoxins having a micellar structure is not less than 300 kDa nor more than 1,000 kDa. The first hollow fiber membrane 16a has first pores 22a with diameters that correspond to this molecular weight. The second hollow fiber membrane 16b adsorbs IL-6 by means of the second pores 22b. IL-6 is a type of inflammatory cytokine. The molecular weight of IL-6 is not less than 20 kDa nor more than 30 kDa. The second hollow fiber membrane 16b has second pores 22a with diameters that correspond to this molecular weight.

Regarding the adsorption characteristics of the endotoxins, the first hollow fiber membrane 16a has a sieving coefficient of not less than 0.12 nor more than 0.28 for dextran having a molecular weight of not less than 300 kDa nor more than 1000 kDa. This sieving coefficient is realized when the first hollow fiber membrane 16a has the first pores 22a. Regarding the adsorption characteristics of IL-6, the second hollow fiber membrane 16b has a sieving coefficient of not less than 0.64 nor more than 0.80 for dextran having a molecular weight of not less than 20 kDa nor more than 30 kDa. This sieving coefficient is realized when the second hollow fiber membrane 16b has the second pores 22b.

The first hollow fiber membrane 16a in the present embodiment comes in contact with blood and adsorbs endotoxins in the blood. The second hollow fiber membrane 16b also comes in contact with blood and adsorbs IL-6 in the blood. Being provided with the hollow fiber membrane bundle 4 in which the first hollow fiber membrane 16a and the second hollow fiber membrane 16b are mixed, the hollow fiber membrane module 1 allows for efficient collection of the endotoxins and IL-6 so as to purify blood.

The first and second hollow fiber membranes 16a and 16b can be produced as follows. In other words, a composite resin containing a polyarylate resin represented by the above chemical formula (1) and a polyethersulfone resin represented by the above chemical formula (2) or (3) is dissolved in a predetermined organic solvent to prepare a spinning dope. The mass ratio of the polyarylate resin to the polyethersulfone resin is, for example, 2:1 to 1:2 for both the first and second hollow fiber membranes 16a and 16b. The organic solvent is not particularly limited as long as the organic solvent can dissolve a polyarylate resin and a polyethersulfone resin. Examples include, e.g., tetrahydrofuran, dioxane, dimethylformamide, dimethylacetamide, N-methyl-2-pyrrolidone, etc. The mass ratio of the composite resin to the organic solvent is, for example, 0.5:9.5 to 3:7. The temperature at which the composite resin is dissolved in the organic solvent is, for example, not less than 30 degrees Celsius nor more than 100 degrees Celsius.

By using a double nozzle to push out the spinning dope together with an internal coagulation liquid and drop the spinning dope and the internal coagulation liquid into a tank containing an external coagulation liquid, a first hollow fiber membrane 16a and a second hollow fiber membrane 16b are formed. The internal coagulation liquid and the

external coagulation liquid are both mixtures of water and an organic solvent. The mixing ratio of water and the organic solvent in the internal coagulation liquid and in the external coagulation liquid is, for example, 4:6 to 7:3. The sieving coefficient of the hollow fiber membranes with respect to the solute can be adjusted by changing the mixing ratio of water and the organic solvent in the internal coagulation liquid. The higher the mixing ratio of the organic solvent in the internal coagulant coagulation liquid, the higher the sieving coefficient of the resulting hollow fiber membranes. Spinning under these conditions allows for the acquisition of a first hollow fiber membrane 16a with first pores 22a having a pore diameter suitable for adsorption of endotoxins and a second hollow fiber membrane 16b with second pore 22b having a pore diameter suitable for adsorption of IL-6.

<Hollow fiber membrane physical properties evaluation>

(Preparation of hollow fiber membrane module 1)

In order to evaluate the physical properties of the hollow fiber membranes 16 with respect to the adsorption of substances, a hollow fiber membrane module 1 filled with three types of hollow fiber membranes 16 with different permeability properties was prepared. Each hollow fiber membrane 16 was prepared according to the procedure described above. In the following, the three hollow fiber membranes 16 are respectively referred to as PEPA-A, PEPA-B, and PEPA-C.

(Preparation of molecular weight cut off curves using dextran)

In order to understand the characteristics of each of the hollow fiber membranes 16 with respect to the adsorption of substances, molecular weight cut off curves of the respective hollow fiber membrane 16 were prepared using dextran. The molecular weight cut off curves were prepared according to a publicly-known method. FIG. 4 is a schematic diagram of a circulator used in a test.

More specifically, various types of dextran were first mixed and diluted to 500 mL with distilled water so as to prepare a dextran solution 24. This solution was dispensed into a container 26 with a volume of 100 mL and used for the test. As the dextran, 0.125 g of dextran with a mass average molecular weight of 9,000-11,000 (model D9260 manufactured by Sigma-Aldrich), 0.125 g of dextran with a mass average molecular weight of 35,000-45,000 (model D1662 manufactured by Sigma-Aldrich), 0.5 g of dextran with a mass average molecular weight of 150,000 (model D4876 manufactured by Sigma-Aldrich), and 0.75 g of dextran with a mass average molecular weight of 180,000-210,000 (model 043-22611 manufactured by FUJIFILM Wako Pure Chemical Corporation) were used.

A circulation process of repeatedly letting the dextran solution 24 flow to PEPA-A through PEPA-C was carried out using a circulator 28 shown in FIG. 4. In FIG. 4, "Bi" indicates an inlet for the solution, "Bo" indicates an outlet for the solution, and "F" indicates an outlet for filtrate. A solution inlet Bi is located at one end of a solution inlet channel 30 and is connected to the container 26. The other end of the solution inlet channel 30 is connected to a blood introduction port 18 of the hollow fiber membrane module 1. For the hollow fiber membrane module 1, PEPA-A through PEPA-C are used, each of which is housed separately. A solution outlet Bo is located at one end of a solution outlet channel 32 and is connected to the container 26. The other end of the solution outlet channel 32 is connected to a blood discharge port 20 of each hollow fiber membrane module 1. A filtrate outlet F is located at one end of a filtrate channel 34 and is connected to the container 26. The other end of the filtrate channel 34 is connected to a filtrate discharge port 36 of the hollow fiber membrane module 1.

An introduction pump 38 is provided in the solution inlet channel 30. A discharge pump 40 is provided in the filtrate channel 34. When the introduction pump 38 is driven, the dextran solution 24 in the container 26 flows through the solution inlet channel 30 and the blood introduction port 18 from the solution inlet Bi and flows into the hollow fiber membrane module 1. The dextran solution 24 that has flown into the hollow fiber membrane module 1 passes through the hollow fiber membranes 16 and then through the blood discharge port 20 and the solution outlet channel 32 and returns from the solution outlet port Bo to the container 26. When the discharge pump 40 is driven, some of the dextran solution 24 passing through the inside of the hollow fiber membranes 16 moves from the inside of the hollow fiber membranes 16 to the outside of the hollow fiber membranes 16. The dextran solution 24 that has moved to the outside of the hollow fiber membranes 16 passes through the filtrate discharge port 36 and the filtrate channel 34 and returns to the container 26 through the filtrate outlet F.

The introduction pump 38 was driven such that the flow rate of the dextran solution 24 in the solution inlet channel 30 was 1.0 mL/min, and the discharge pump 40 was driven such that the flow rate of the filtrate in the filtrate channel 34 was 0.1 mL/min. After two hours had elapsed from the start of driving each pump, the solution passing through each of the solution inlet Bi, the solution outlet Bo, and the filtrate outlet F was sampled.

The molecular weight of dextran contained in each sampled solution was measured by high performance liquid chromatography (HPLC). The high performance liquid chromatography was performed using a 2695 separation module

(manufactured by Waters), a 2414 differential refractive index detector (manufactured by Waters), and two separation columns GF-710HQ (manufactured by Shodex). The measurement conditions were as follows: flow rate of 0.6 mL/min, a column temperature of 40 degrees Celsius, an injection volume of 100 uL, and a measurement time of 60 minutes. The two separation columns were connected in series.

From the results obtained, the continuous sieving coefficient (SC) for each retention time was calculated. The sieving coefficient is an index of the filtration ratio of a solute. SC is equal to one (SC = 1) if all the solute is filtered, and SC is equal to zero (SC = 0) if no solute is filtered. The sieving coefficient can be calculated using the following mathematical formula (3). In mathematical formula (3), CF represents the refractive index intensity of a sample taken at the filtrate outlet F. CBi represents the refractive index intensity of a sample taken at the solution inlet Bi. CBo represents the refractive index intensity of a sample taken at the solution outlet Bo.

$$SC = 2CF/(CBi + CBo) \qquad (3)$$

From the linear function of a calibration curve obtained by performing high performance liquid chromatography on the dextran solution 24, the retention time of dextran of each molecular weight in each hollow fiber membrane 16 was converted to a peak top molecular weight (Mp). The results for the respective hollow fiber membranes 16 were then plotted on a graph with SC on the vertical axis and the logarithm of Mp on the horizontal axis so as to obtain molecular weight cut off curves of the respective hollow fiber membranes 16. FIG. 5A shows the molecular weight cut off curves of the respective hollow fiber membranes.

Based on the obtained molecular weight cut off curves, sieving coefficients of the respective hollow fiber membranes in each of the molecular weight range of the endotoxins and the molecular weight range of IL-6 were derived. FIG. 5B shows the sieving coefficients of the respective hollow fiber membranes in the molecular weight range of endotoxins and the molecular weight range of IL-6. As shown in FIG. 5B, the sieving coefficient of PEPA-A in the molecular weight range of the endotoxins was not less than 0.12 nor more than 0.28. The sieving coefficient of PEPA-C in the molecular weight range of IL-6 was not less than 0.64 nor more than 0.80. Therefore, PEPA-A corresponds to the first hollow fiber membrane 16a, and PEPA-C corresponds to the second hollow fiber membrane 16b.

<Evaluation of endotoxin adsorption ability of hollow fiber membranes>

In order to evaluate the endotoxin adsorption ability of each hollow fiber membrane 16, an endotoxin adsorption test explained below was conducted using each hollow fiber membrane 16.

(Preparation of endotoxin solution)

First, 0.02 mL of endotoxins (1,000 EU/mL) was diluted with 20 mL of pyrogen-free water (model H20CC0124, manufactured by Merck) so as to prepare an endotoxin solution (1 EU/mL). For the endotoxin (1,000 EU/mL), an endotoxin standard (10,000 EU/mL, manufactured by Pharmaceutical and Medical Device Regulatory Science Society of Japan) diluted 10-fold with pyrogen-free water was used. The prepared endotoxin solution was put in a dry heat sterilized glass container. In addition, several glass containers containing 20 mL of the endotoxin solution were prepared.

A circulation process of repeatedly letting the endotoxin solution flow to PEPA-A through PEPA-C was carried out using the circulator 28 shown in FIG. 4. For the container 26, the above-described glass container containing 20 mL of the endotoxin solution was used. An autoclavesterilized silicone tube was used for each channel. PEPA-A through PEPA-C were gamma-sterilized for use.

The introduction pump 38 was driven such that the flow rate of the endotoxin solution in the solution inlet channel 30 was 1.0 mL/min, and the discharge pump 40 was driven such that the flow rate of the filtrate in the filtrate channel 34 was 0.1 mL/min. At each of time points of 0, 10, 30, 60, and 120 minutes after the start of the driving of each pump, the endotoxin solution in the container 26 was sampled. Endotoxin-free pipette tips and dry heat sterilized glass containers were used for the sampling.

Endospecy ES-50M (manufactured by Seikagaku Corporation) and PyroColor Diazo Reagents DIA150-MP (manufactured by Seikagaku Corporation) were used to measure the endotoxin concentration of each sampled solution using the endpoint colorimetric method. The measurement procedure was performed according to the protocol of a reagent set. Each sampled solution was diluted 100-fold with pyrogen-free water. Microplates (model 900570, manufactured by Seikagaku Corporation) and pipette tips (model 298-35031, 291-35021, manufactured by FUJIFILM Wako Pure Chemical Corporation) that were used were all endotoxin-free. The absorbance of each solution was measured using a microplate reader (Wallac 1420 ARVO MX, manufactured by PerkinElmer) at a measurement wavelength of 540 nm and a control wavelength of 630 nm. The circulation process and concentration measurement were performed three times, and the average of the concentrations obtained in the respective measurements was calculated.

As a control plot, the circulation process and concentration measurement were performed in the same manner except that the hollow fiber membrane module 1 was not attached to the circulator 28 and that the solution inlet channel 30 was directly connected to the solution outlet channel 32 and the filtrate channel 34.

FIGS. 6A to 6D are diagrams showing the results of endotoxin adsorption tests. FIG. 6A shows the result of PEPA-A, FIG. 6B shows the result of PEPA-B, FIG. 6C shows the result of PEPA-C, and FIG. 6D shows the result of the control plot. As shown in FIGS. 6A to 6D, a significant decrease in the endotoxin concentration was confirmed in PEPA-A. In PEPA-B and PEPA-C, the decrease in the endotoxin concentration was small. Since the circulator 28 has a channel structure where an endotoxin solution and a filtrate that have passed through the hollow fiber membrane module 1 go back to the container 26, a decrease in the endotoxin concentration means that the endotoxin has been adsorbed and removed by the hollow fiber membranes 16.

Further, for PEPA-A to PEPA-C and the control plot, the adsorption amount of endotoxin (M) after the circulation process was performed for 120 minutes was calculated using the following mathematical formula (4). In mathematical formula (4), $CB_0$ represents the endotoxin concentration in the endotoxin solution before the start of the circulation process. $CB_{120}$ represents the endotoxin concentration in the endotoxin solution after the circulation process was performed for 120 minutes. 20 represents the volume of the endotoxin solution.

$$M = (CB_0 - CB_{120}) \times 20 \qquad (4)$$

FIG. 7 is a diagram showing the calculation results of endotoxin adsorption amounts. As shown in FIG. 7, the adsorption amount of endotoxins was significantly higher in PEPA-A than those in PEPA-B and PEPA-C. From the above, it has been shown that the endotoxins can be efficiently adsorbed and removed by the first hollow fiber membrane 16a having a sieving coefficient of not less than 0.12 nor more than 0.28 for dextran having a molecular weight of not less than 300 kDa nor more than 1000 kDa.

<Evaluation of IL-6 adsorption ability of hollow fiber membranes>

In order to evaluate the IL-6 adsorption ability of each hollow fiber membrane 16, an IL-6 adsorption test explained below was conducted using each hollow fiber membrane 16.

(Preparation of IL-6 solution)

First, an IL-6 solution (10,000 pg/mL) containing 18 mL of PBS (model 045-29795, manufactured by FUJIFILM Wako Pure Chemical Corporation), 2 mL of Blocker (registered trademark) BSA (10×) in PBS (Model 37525, manufactured by Thermo Fisher), and 0.02 mL of human recombinant IL-6 (IL-6, Human, Recombinant, 10 μg/mL, model 206-IL-010, manufactured by R&D Systems) was prepared. The human recombinant IL-6 prepared to 10 ug/mL using 1% Blocker (registered trademark) BSA/PBS was used. Several containers containing 20 mL of the prepared IL-6 solution were prepared.

A circulation process of repeatedly letting the IL-6 solution flow to PEPA-A through PEPA-C was carried out using the circulator 28 shown in FIG. 4. For the container 26, the above-described container containing 20 mL of the IL-6 solution was used. The introduction pump 38 was driven such that the flow rate of the IL-6 solution in the solution inlet channel 30 was 1.0 mL/min, and the discharge pump 40 was driven such that the flow rate of the filtrate in the filtrate channel 34 was 0.1 mL/min. At each of time points of 0, 10, 30, 60, and 120 minutes after the start of the driving of each pump, the IL-6 solution in the container 26 was sampled.

A human IL-6 quantification kit (Human IL-6 Quantikine ELISA Kit, model D6050, manufactured by R&D systems) was used to measure the IL-6 concentration in each sampled solution. The measurement procedure was performed according to the protocol of the quantification kit. Each sampled solution was diluted 100-fold with a dilution buffer (Calibrator Diluent, model RD5T) attached to the kit. The absorbance of each solution was measured using a microplate reader (Wallac 1420 ARVO MX, manufactured by PerkinElmer) at a measurement wavelength of 450 nm and a control wavelength of 540 nm. The circulation process and concentration measurement were performed three times, and the average of the concentrations obtained in the respective measurements was calculated.

As a control plot, the circulation process and concentration measurement were performed in the same manner except that the hollow fiber membrane module 1 was not attached to the circulator 28 and that the solution inlet channel 30 was directly connected to the solution outlet channel 32 and the filtrate channel 34.

FIGS. 8A to 8D are diagrams showing the results of IL-6 adsorption tests. FIG. 8A shows the result of PEPA-A, FIG. 8B shows the result of PEPA-B, FIG. 8C shows the result of PEPA-C, and FIG. 8D shows the result of the control plot. As shown in FIGS. 8A to 8D, a significant decrease in the IL-6 concentration was confirmed in PEPA-C. In PEPA-A and PEPA-B, the decrease in the endotoxin concentration was small. Since the circulator 28 has a channel structure

where an IL-6 solution and a filtrate that have passed through the hollow fiber membrane module 1 go back to the container 26, a decrease in the IL-6 concentration means that IL-6 has been adsorbed and removed by the hollow fiber membranes 16.

Further, for PEPA-A to PEPA-C and the control plot, the adsorption amount of IL-6 (M) after the circulation process was performed for 120 minutes was calculated using the above mathematical formula (4). In mathematical formula (4), $CB_0$ represents the IL-6 concentration in the IL-6 solution before the start of the circulation process. $CB_{120}$ represents the IL-6 concentration in the IL-6 solution after the circulation process was performed for 120 minutes. 20 represents the volume of the IL-6 solution.

FIG. 9 is a diagram showing the calculation results of IL-6 adsorption amounts. As shown in FIG. 9, the adsorption amount of IL-6 was significantly higher in PEPA-C than those in PEPA-A and PEPA-B. From the above, it has been shown that IL-6 can be efficiently adsorbed and removed by the second hollow fiber membrane 16b having a sieving coefficient of not less than 0.64 nor more than 0.80 for dextran having a molecular weight of not less than 20 kDa nor more than 30 kDa.

As explained above, the hollow fiber membrane module 1 according to the present embodiment has a first hollow fiber membrane 16a whose sieving coefficient for dextran having a molecular weight of not less than 300 kDa nor more than 1000 kDa is not less than 0.12 nor more than 0.28, and the first hollow fiber membrane 16a adsorbs the endotoxins. Thereby, simply by incorporating the hollow fiber membrane module 1 into a channel of a target object from which the endotoxins are to be removed, the endotoxins can be adsorbed and removed from the target object. Therefore, compared to a case where a conventional immobilized polymyxin molded article is used, the complication of the structure of a blood circuit and an increase in the cost of treatment can be suppressed. Further, since the hollow fiber membrane module 1 adsorbs the endotoxins by means of the first hollow fiber membrane 16a, a task of fixing an endotoxin adsorbent, ligand, or the like to the carrier is not necessary. Therefore, the present embodiment can provide a more practical endotoxin removal technology.

The first hollow fiber membrane 16a according to the present embodiment adsorbs the endotoxins in the blood. In other words, the hollow fiber membrane module 1 is used as a blood purifier. This allows for more efficient blood purification.

Further, the first hollow fiber membrane 16a according to the present embodiment is composed of a polyester polymer alloy membrane containing a polyarylate resin represented by the above chemical formula (1) and a polyethersulfone resin represented by the above chemical formula (2) or (3). In this case, two types of hydrophobic polymers, i.e., polyarylate and polyethersulfone, shrink to different degrees to form pores 22 of a desired diameter. Therefore, there is no need to use an opening agent of a hydrophilic polymer such as polyvinylpyrrolidone to form the pores 22. Therefore, the first hollow fiber membrane 16a can be manufactured more easily.

Also, the hollow fiber membrane module 1 of the present embodiment has a second hollow fiber membrane 16b whose sieving coefficient for dextran having a molecular weight of not less than 20 kDa nor more than 30 kDa is not less than 0.64 nor more than 0.80, and the second hollow fiber membrane 16b adsorbs IL-6. This allows for simultaneous adsorption and removal of both the endotoxins and IL-6. Thus, a more practical endotoxin removal technology can be provided.

Described above is a detailed explanation on the embodiment of the present invention. The above-described embodiments merely show specific examples for carrying out the present invention. The details of the embodiment do not limit the technical scope of the present invention, and many design modifications such as change, addition, deletion, etc., of the constituent elements may be made without departing from the spirit of the present invention defined in the claims. New embodiments resulting from added design change will provide the advantages of the embodiments and variations that are combined. In the above-described embodiments, the details for which such design change is possible are emphasized with the notations "according to the embodiment", "in the embodiment", etc. However, design change is also allowed for those without such notations. Optional combinations of the above components are also valid as embodiments of the present invention.

The inventions according to the above-described embodiments may be defined by the items described in the following.

[Item 1]

A method for removing endotoxins, including bringing a hollow fiber membrane module (1) according to an embodiment to come into contact with the endotoxins.

[Item 2]

A blood purifier including the hollow fiber membrane module (1) according to the embodiment.

[INDUSTRIAL APPLICABILITY]

The present invention can be used for hollow fiber membrane modules and a method for removing endotoxins.

[REFERENCE SIGNS LIST]

1 hollow fiber membrane module

16 hollow fiber membrane
16a first hollow fiber membrane
16b second hollow fiber membrane
22 pore
22a first pore
22b second pore

## Claims

1. A hollow fiber membrane module comprising:

   a first hollow fiber membrane having a sieving coefficient of not less than 0.12 nor more than 0.28 for dextran having a molecular weight of not less than 300 kDa nor more than 1000 kDa, wherein
   the first hollow fiber membrane adsorbs endotoxins.

2. The hollow fiber membrane module according to claim 1, wherein the first hollow fiber membrane adsorbs the endotoxins in blood.

3. The hollow fiber membrane module according to claim 1 or 2, wherein

   the first hollow fiber membrane is composed of a polyester polymer alloy membrane containing a polyarylate resin having repeating units represented by the following chemical formula (1) and a polyethersulfone resin having repeating units represented by the following chemical formula (2) or (3):

   [Chemical 1]

   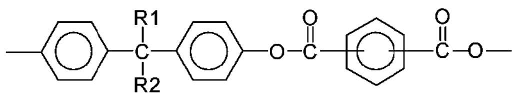

   (1)

   where, in the chemical formula (1), R1 and R2 are lower alkyl groups having 1 to 5 carbons, and R1 and R2 may be identical or different from each other;

   [Chemical 2]

   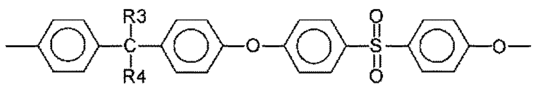

   (2)

   where, in the chemical formula (2), R3 and R4 are lower alkyl groups having 1 to 5 carbons, and R3 and R4 may be identical or different from each other; and

[Chemical 3]

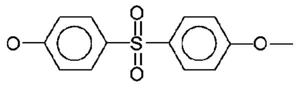 (3)

4. The hollow fiber membrane module according to any one of claims 1 through 3, comprising:

a second hollow fiber membrane having a sieving coefficient of not less than 0.64 nor more than 0.80 for dextran having a molecular weight of not less than 20 kDa nor more than 30 kDa, wherein
the second hollow fiber membrane adsorbs interleukins 6.

5. A method for removing endotoxins, comprising:
bringing the hollow fiber membrane module according to any one of claims 1 through 4 to come into contact with the endotoxins.

FIG. 1

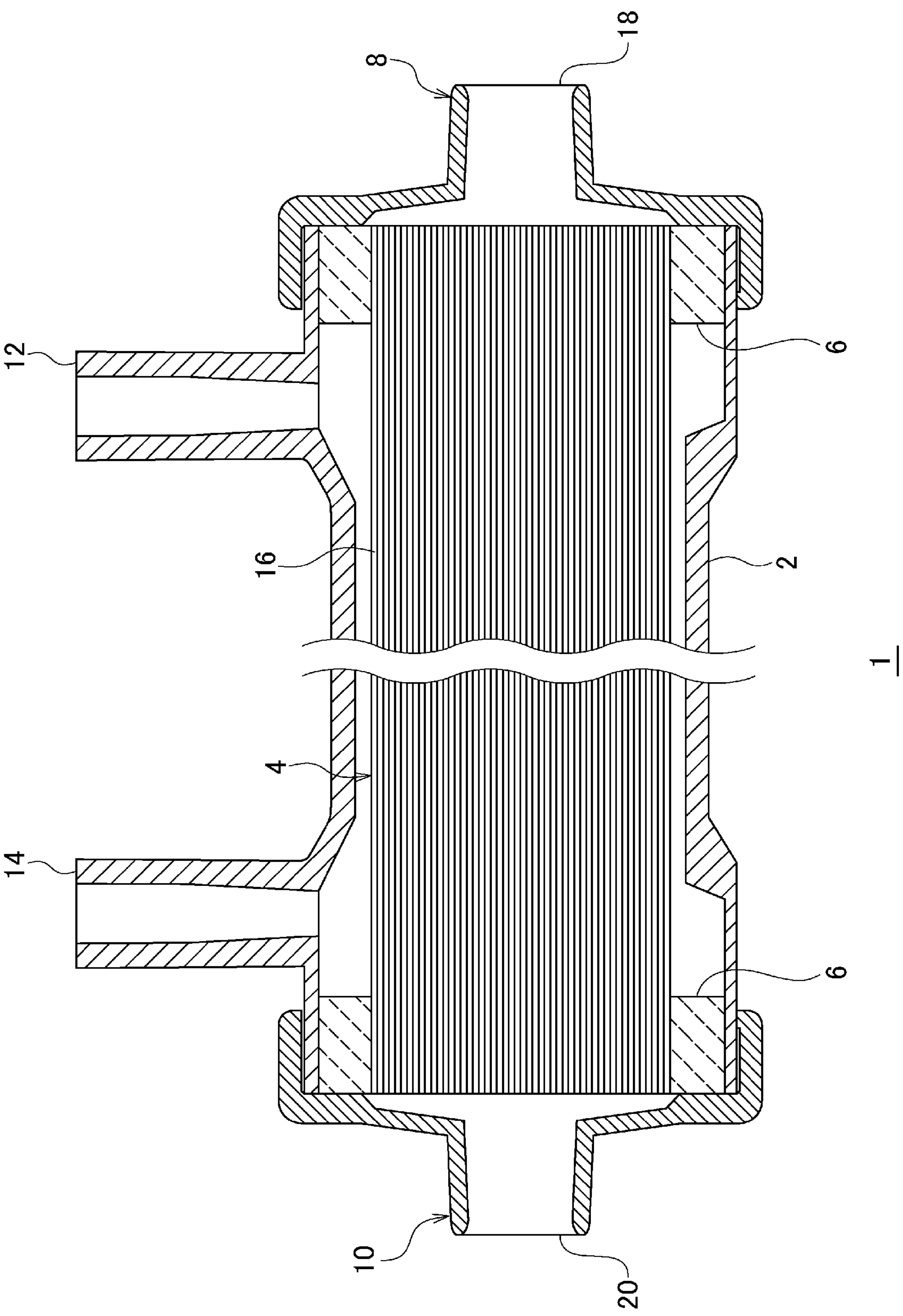

FIG. 2

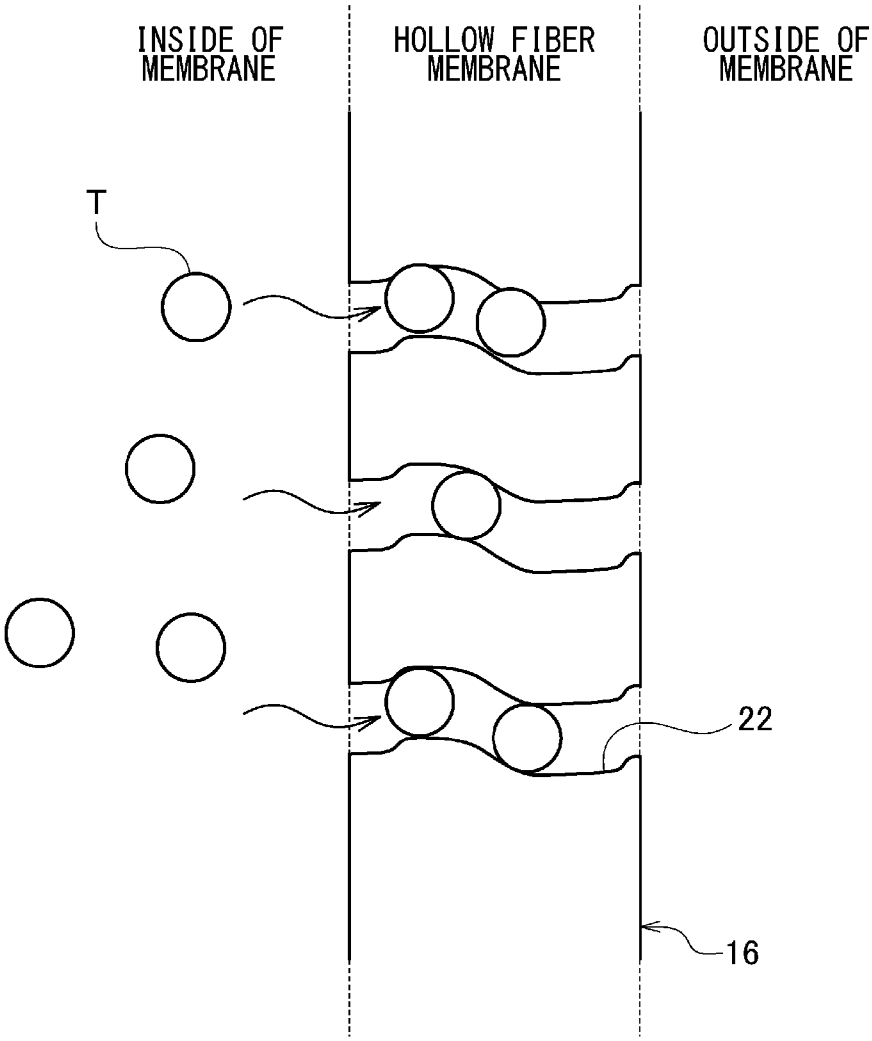
INSIDE OF MEMBRANE
HOLLOW FIBER MEMBRANE
OUTSIDE OF MEMBRANE
T
22
16

FIG. 3A

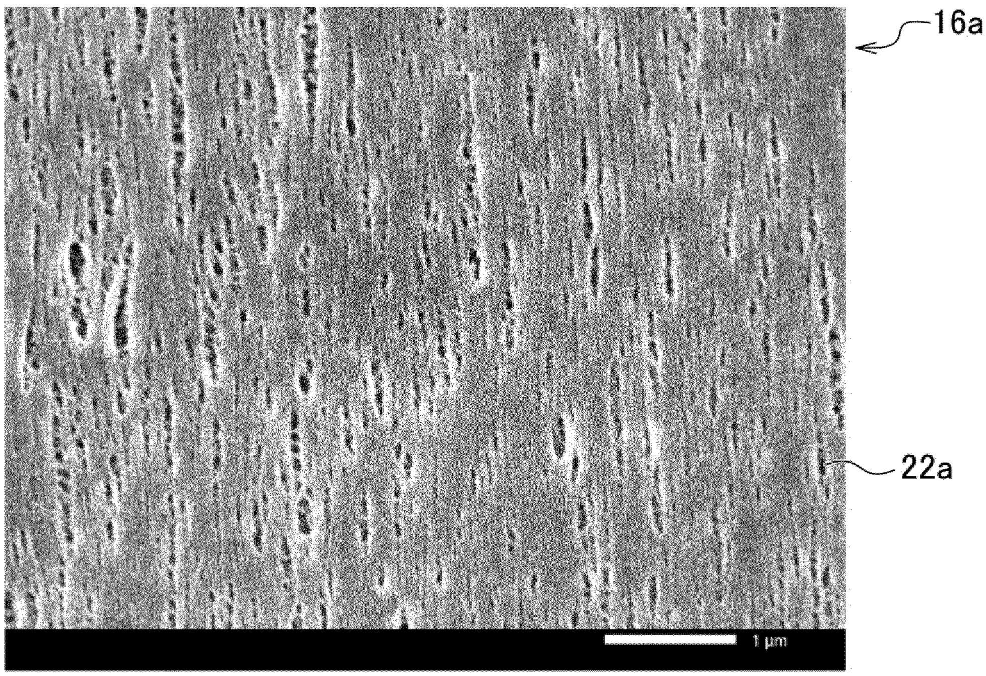


FIG. 3B

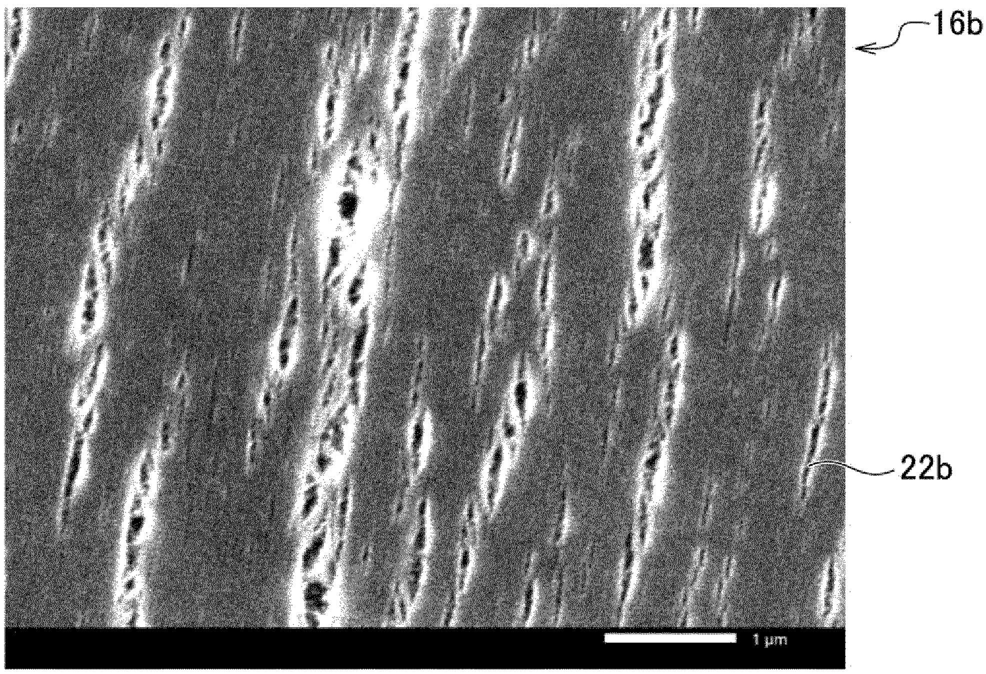

FIG. 4

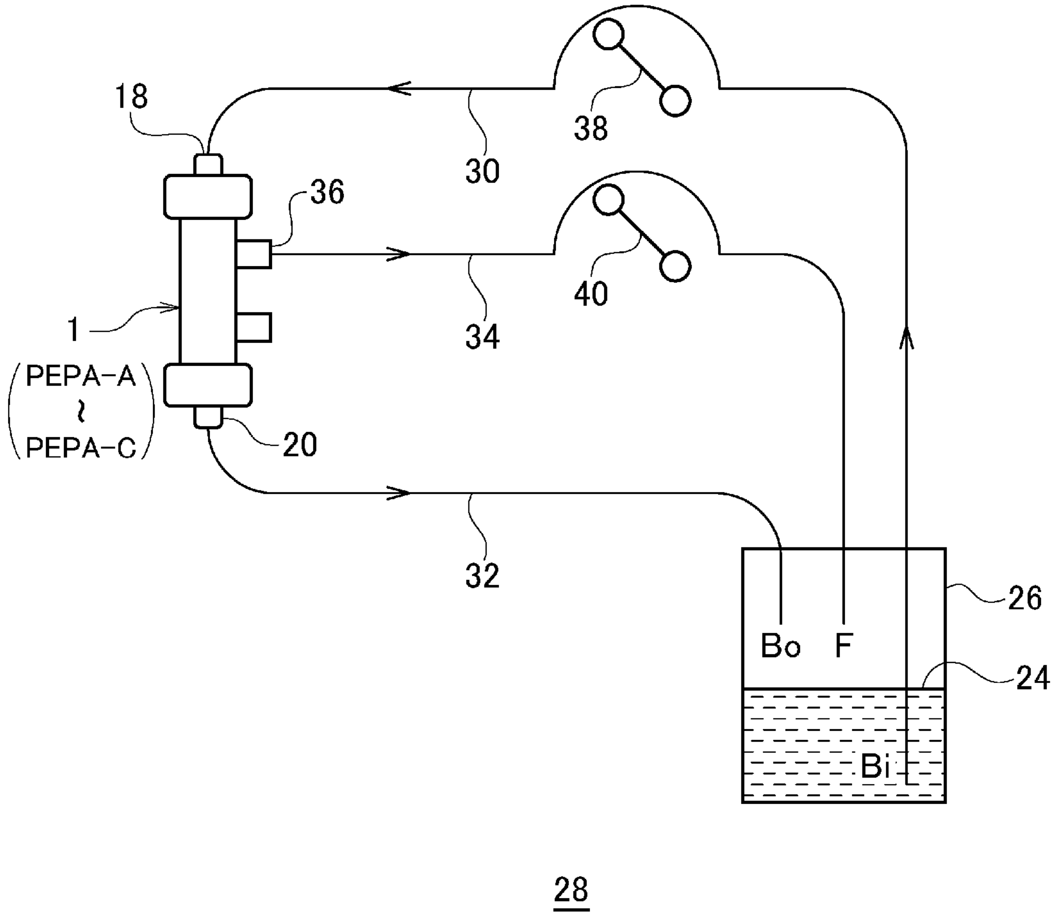

FIG. 5A

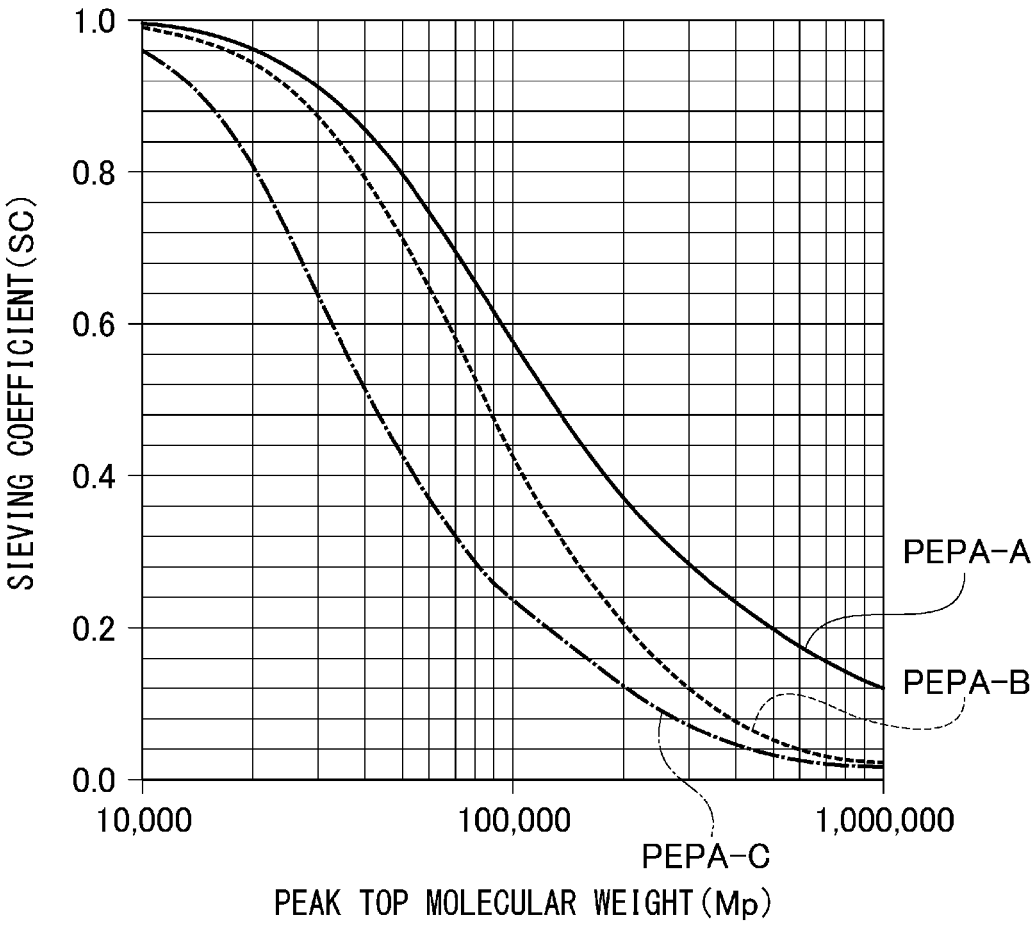


FIG. 5B

| | 300-1,000 kDa (ENDOTOXIN MICELLE RANGE) SIEVING COEFFICIENT (SC) | 20-30 kDa (IL-6 RANGE) SIEVING COEFFICIENT (SC) |
|---|---|---|
| PEPA-A | 0.12～0.28 | 0.92～0.96 |
| PEPA-B | 0.01～0.12 | 0.88～0.93 |
| PEPA-C | 0.01～0.08 | 0.64～0.80 |

FIG. 6A

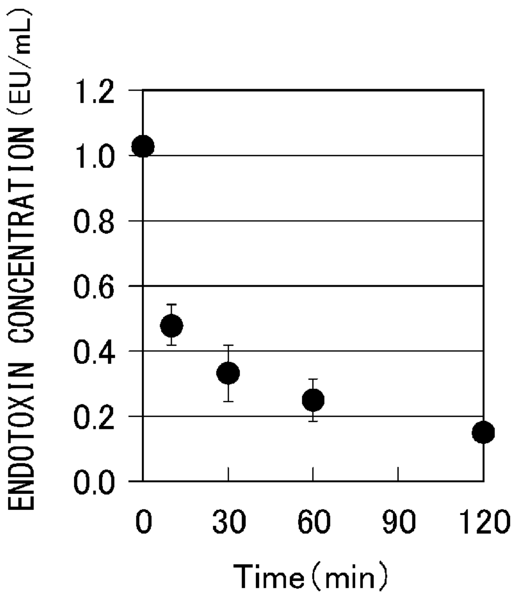


FIG. 6B

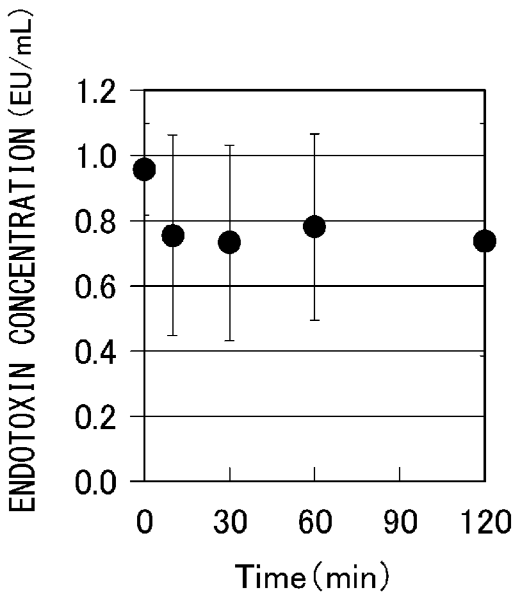


FIG. 6C

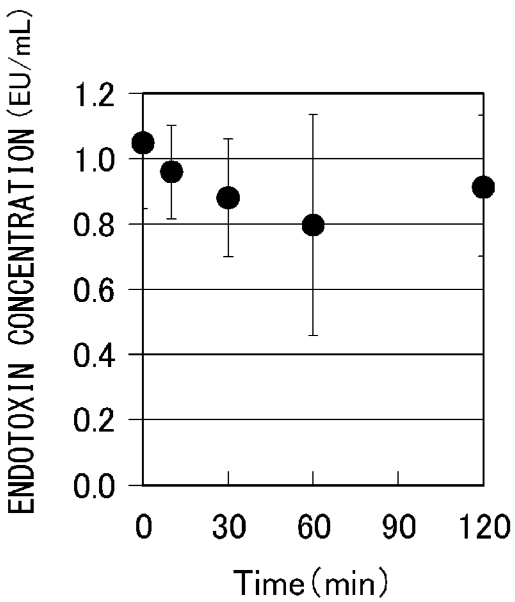


FIG. 6D

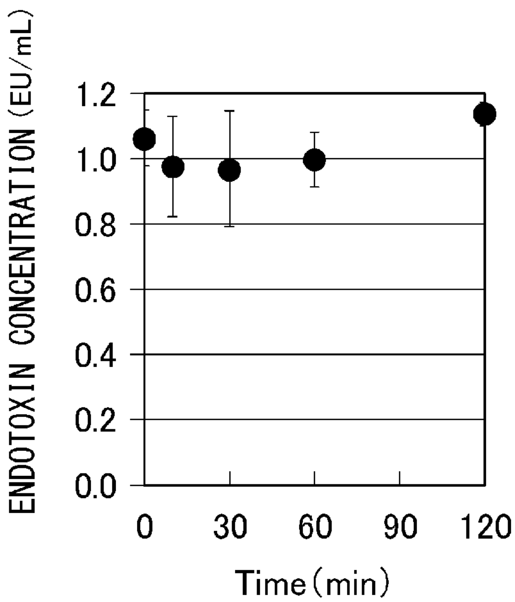

FIG. 7

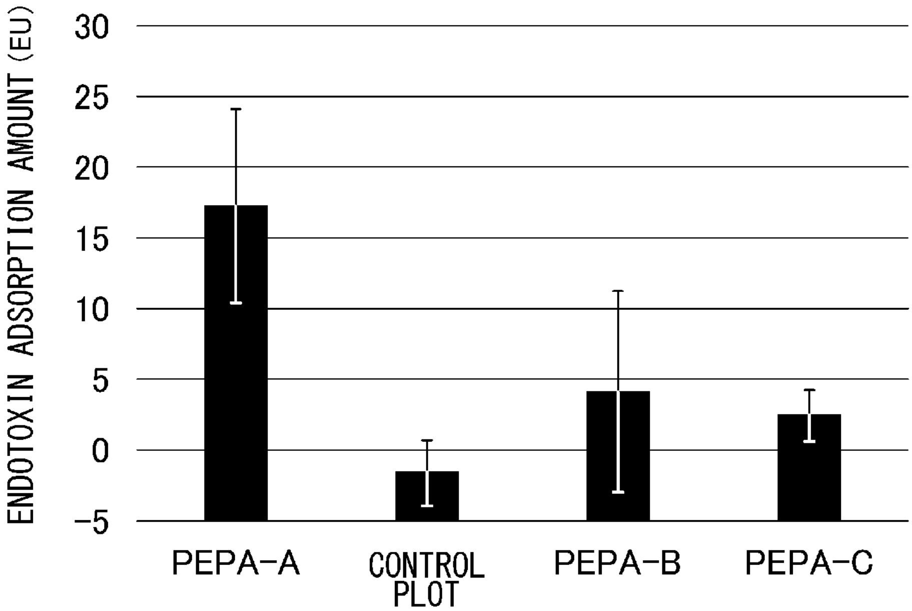
ENDOTOXIN ADSORPTION AMOUNT (EU)
30
25
20
15
10
5
0
−5
PEPA-A
CONTROL PLOT
PEPA-B
PEPA-C

FIG. 8A

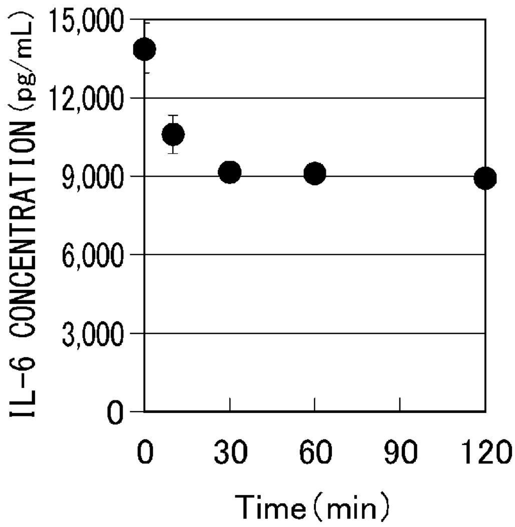


FIG. 8B

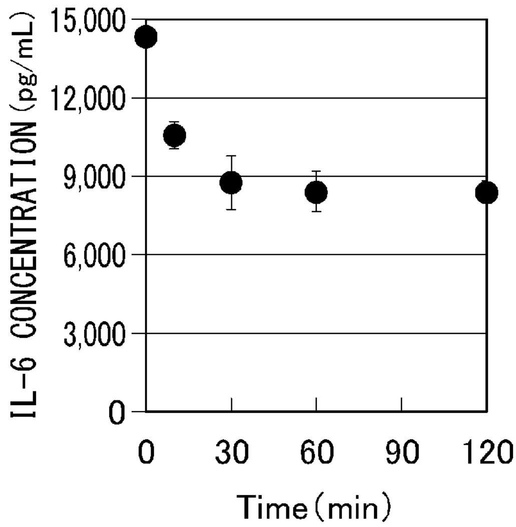


FIG. 8C

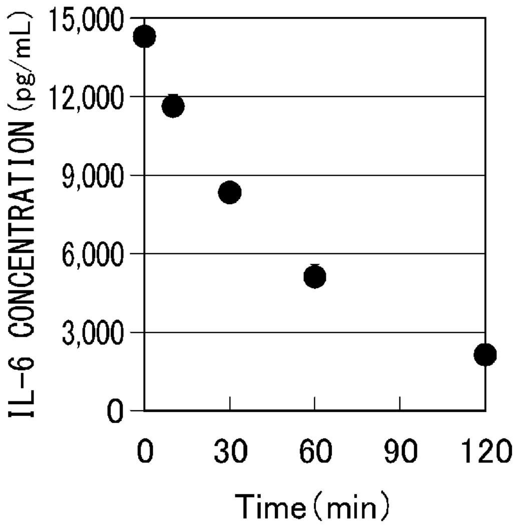


FIG. 8D

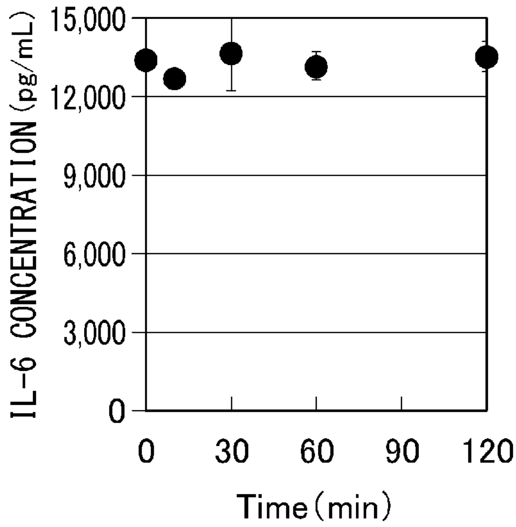

FIG. 9

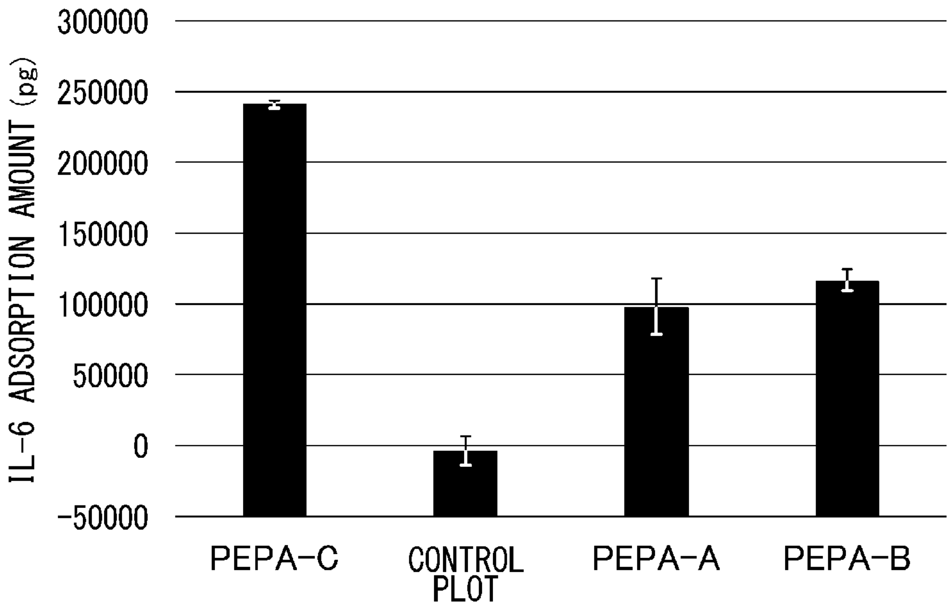
IL-6 ADSORPTION AMOUNT (pg)
300000
250000
200000
150000
100000
50000
0
-50000
PEPA-C
CONTROL PLOT
PEPA-A
PEPA-B

**INTERNATIONAL SEARCH REPORT**

International application No.
PCT/JP2021/016934

A. CLASSIFICATION OF SUBJECT MATTER
Int. Cl. B01D61/14(2006.01)i, B01D63/02(2006.01)i, B01D71/48(2006.01)i, B01D71/68(2006.01)i, B01J20/26(2006.01)i, A61M1/18(2006.01)i
FI: A61M1/18 500, B01D63/02, B01D71/48, B01D71/68, B01D61/14 500, B01J20/26 H
According to International Patent Classification (IPC) or to both national classification and IPC

B. FIELDS SEARCHED
Minimum documentation searched (classification system followed by classification symbols)
Int. Cl. B01D61/14, B01D63/02, B01D71/48, B01D71/68, B01J20/26, A61M1/18

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 2008-173163 A (NIKKISO CO., LTD.) 31 July 2008, paragraphs [0021], [0031]-[0034], [0038], fig. 1, 4 | 1-5 |
| Y | JP 10-57476 A (TORAY INDUSTRIES, INC.) 03 March 1998, paragraphs [0001], [0011], [0020], table 2 | 1-5 |
| Y | JP 2017-510435 A (GAMBRO LUNDIA AB) 13 April 2017, paragraphs [0002], [0019], fig. 4 | 4-5 |

☐ Further documents are listed in the continuation of Box C.
☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02.06.2021 | 15.06.2021 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2021/016934

| Patent Documents referred to in the Report | Publication Date | Patent Family | Publication Date |
|---|---|---|---|
| JP 2008-173163 A | 31.07.2008 | (Family: none) | |
| JP 10-57476 A | 03.03.1998 | (Family: none) | |
| JP 2017-510435 A | 13.04.2017 | US 2017/0165616 A1<br>paragraphs [0002], [0018], fig. 4<br>WO 2015/118045 A1<br>CN 105722583 A<br>KR 10-2016-0118344 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP S61135674 A